# FASCICULE DE BREVET EUROPEEN

(11) **EP 1 042 463 B1**
(45) Date de publication et mention de la délivrance du brevet: **29.09.2004**
(21) Numéro de dépôt: 98964526.2
(22) Date de dépôt: 28.12.1998
(51) Int. Cl.: C12N 15/11, A61K 31/70, C12N 9/78, C07K 14/47, G01N 33/50, C12Q 1/68

(54) **CIBLE DE TRAITEMENT DE L'ATHEROSCLEROSE, DE L'OBESITE ET DU DIABETE DE TYPE II**
TARGET ZUR BEHANDLUNG VON ATHEROSKLEROSE, FETTLEIBIGKEIT UND DIABETIS TYP II
TARGET FOR TREATING ATHEROSCLEROSIS, OBESITY AND TYPE II DIABETES

(30) Priorité: 30.12.1997 FR 9716655
(43) Date de publication de la demande: 11.10.2000
(73) Titulaire: OBE THERAPY BIOTECHNOLOGY, 91000 Evry (FR)
(72) Inventeur: Harosh, Itzik, 75014 Paris (FR)
(74) Mandataire: Desaix, Anne
(86) Numéro de dépôt international: PCT/FR1998/002883
(87) Numéro de publication internationale: WO 1999/035257

(56) Documents cités:
- WO-A-98/45472
- LAU P P ET AL: "Cloning of an Apobec -1-binding protein that also interacts with apolipoprotein B mRNA and evidence for its involvement in RNA editing." JOURNAL OF BIOLOGICAL CHEMISTRY, (1997 JAN 17) 272 (3) 1452-5., XP002076612 cité dans la demande
- PHUNG, T. ET AL.: "Regulation of hepatic Apolipoprotein B RNA editing in the genetically obese Zucker rat." METABOLISM, vol. 45, septembre 1996, pages 1056-1058, XP002076613 cité dans la demande
- OKA, K.: "Tissue-specific inhibition of apolipoprotein B mRNA editing in the liver by adenovirus-mediated transfer of a dominant negative mutant APOBEC-1 leads to increased low density lipoprotein in mice." JOURNAL OF BIOLOGICAL CHEMISTRY., vol. 272, 17 janvier 1997, pages 1456-1460, XP002076614
- ANANT, S. ET AL.: "AU-rich RNA binding proteins HelN1 and AUF1 bind apolipoprotein B mRNA and inhibit posttranscriptional C to U editing" NUCLAIC ACIDS SYMPOSIUM SERIES, vol. 36, 1997, pages 115-118, XP002076615
- GREEVE J ET AL: "RECONSTITUTION OF APOB MRNA EDITING TO LIVER BY GENE-TRANSFER AS A POTENTIAL APPROACH FOR THE TREATMENT OF SEVERE FORMS OF POLYGENIC HYPERCHOLESTEROLEMIA - AN OUTLINE OF THE RATIONALE" ZEITSCHRIFT FUER GASTROENTEROLOGIE, vol. 34, no. SUPPL. 03, juin 1996, pages 27-30, XP000652814
- SMITH H C ET AL: "Base-modification mRNA editing through deamination - the good, the bad and the unregulated" TRENDS IN GENETICS, vol. 12, no. 10, octobre 1996, page 418-424 XP004037182

## Description

Cette invention concerne l'enzyme apobec-1 et les protéines associées qui permettent la production de la protéine apoB48 dans l'intestin. L'invention porte notamment sur une méthode de détection d'inhibiteurs d'apobec-1 et des protéines associées, pour un traitement de l'athérosclérose, de l'obésité, du diabète de type **II** (non-insuline dépendant), ou autres maladies caractérisées notamment par un niveau de chylomicrons et/ou de VLDL dans le plasma supérieur à la normale (hyperlipidémies, comme par exemple hypercholestérolémie, hypertriglycéridémie, etc) et/ou par une hyperglicémie.

Le gène apoB code pour deux protéines, apoB100 et apoB48. Ces deux protéines sont traduites à partir du même ARN messager, modifié au niveau d'un unique nucléotide par une enzyme spécialisée, apobec-1 (pour: apoB editing compound 1) et des protéines associées. Dans le système digestif de l'homme, cette enzyme est exprimée dans l'intestin mais pas dans le foie. Dans l'intestin, elle modifie le codon 6666 de l'ARN messager d'apoB en créant un codon stop, qui aboutit à la production d'un polypeptide nommé apoB48 (pour 48 % de l'ARN messager traduit). ApoB48 est essentielle pour la formation des chylomicrons, qui servent à l'absorption et au transport du cholestérol, des triglycérides et autres lipides en provenance de l'intestin. Dans le foie, où l'enzyme apobec-1 n'est pas exprimée, et où aucune modification de l'ARN messager d'apoB n'a lieu, la protéine produite est apoB100, qui fait partie des lipoprotéines de très faible densité (VLDL, Very Low Density Lipoproteins) et de faible densité (LDL, Low Density Lipoproteins).

Il existe actuellement trois maladies génétiques humaines qui affectent le niveau d'expression d'apoB, toutes ayant des phénotypes similaires: l'abétalipoprotéinémie, l'hypobétalipoprotéinémie et la maladie d'Anderson, également appelée maladie de la rétention de chylomicrons. L'abétalipoprotéinémie est due à une déficience en MTP (Microsomal Transfert Protein), alors que l'hypobétaprotéinémie est due à des mutations multiples du gène apoB. Dans ces deux cas, aucun VLDL ni chylomicron n'est détectable dans le plasma, associé respectivement à l'absence d'apoB 100 et d'apoB48.

Par contre, dans la maladie d'Anderson, seuls manquent dans le plasma les chylomicrons (apoB48), tandis que les VLDL (apoB100) restant détectables.

Dans cette invention, il est suggéré:
1. que l'enzyme apobec-1 et ses protéines associées sont des cibles potentielles pour le traitement de l'obésité, et autres maladies caractérisées notamment par un niveau de chylomicrons et/ou de VLDL dans le plasma supérieur à la normale (hyperlipidémies, comme par exemple hypercholestérolémie, hypertriglycéridémie, etc) et/ou par une hyperglicémie.

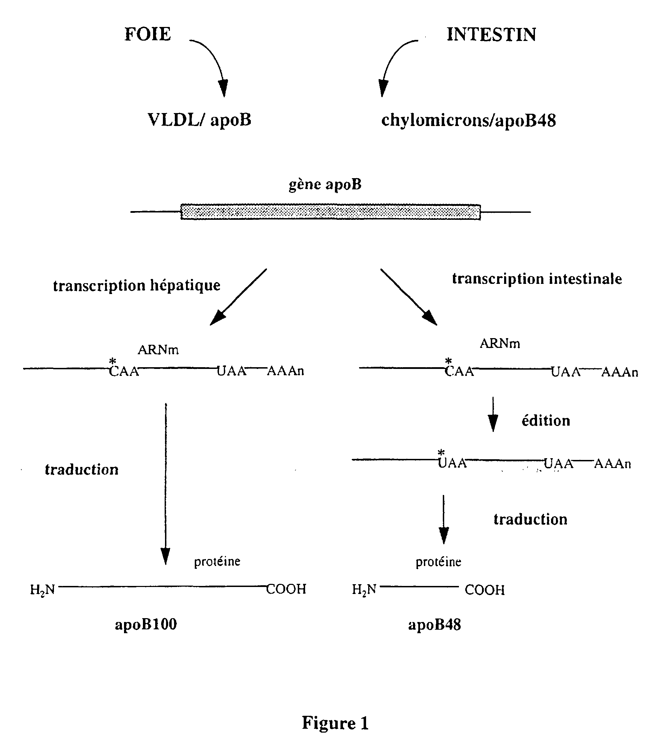

La protéine apoB humaine est la principale apolipoprotéine des lipoprotéines riches en triglycérides (présentes dans les VLDL, LDL et chylomicrons). Son gène est exprimé à la fois dans l'intestin et dans le foie. Le foie produit une protéine de 4536 acides aminés appelée apoB100, tandis que dans l'intestin, le même gène code pour une protéine plus petite qui contient 2152 acides aminés, appelée apoB48. Cette protéine est identique à la partie N-terminale de l'apoB100. ApoB48 est le résultat de la traduction de l'ARN messager (ARNm) d'apoB modifié post-transcriptionnellement par l'enzyme apobec-1 (protéine d'édition de l'ARN messager d'apoB) au niveau du nucléotide 6666 (cytidine), qui subit une déamination donnant une uridine. Cette modification de l'ARN messager d'apoB crée un codon stop (UAA) (voir figure 1 et Chan L (1995) Biochimie, 75-78).

Chez l'homme et le rat, l'ADN complémentaire (ADNc) qui code pour apobec-1 a. été cloné et séquencé récemment (Teng B et al (1993) Science, 1816-1819, Lau PP (1994) Proc. Natl. Acad. Sci. USA, 8522-8526). Chez l'homme, ce gène est exprimé uniquement dans le petit intestin, seul endroit où sont produits apoB48 et les chylomicrons. Dans le foie humain, aucune production d'apoB48 n'a lieu, ce qui est cohérent avec l'absence d'observation de l'enzyme apobec-1 dans cet organe. Au contraire, chez le rat, chez qui apoB100 et apoB48 sont produites dans le foie et l'intestin, apobec-1 est exprimée dans les deux organes, ce qui suggère un rôle essentiel de cette protéine dans la production d'apoB48 (Giannoni et al (1994) J. Biol. Chem., 5932-5936). Par ailleurs, il a été montré par les mêmes auteurs que la transfection de cellules hépatiques HepG2 avec un ADNc d'apobec-1 conduit à une modification de l'ARNm endogène d'apoB, et à la secrétion de la protéine apoB48 (Giannoni et al (1994) J. Biol. Chem., 5932-5936).

Enfin, il a été démontré récemment que des souris transgéniques knock-out pour le gène apobec-1 perdent l'activité d'apobec-1, et ne possèdent aucune trace d'apoB48 dans la circulation sanguine (Hirano KI et al (1996) J. Biol. Chem.7154-7159, Morrison JR et al (1996) Proc. Natl. Acad. Sci. USA, 9887-9890).

Trois maladies humaines d'origine génétique possédant un phénotype semblable ont été décrites: l'abétalipoprotéinémie, l'hypobétalipoprotéinémie et la maladie d'Anderson, également appelée maladie de la rétention de chylomicrons (tableau 1 et Havel RJ and Kane JB (1995) in The metabolic and molecular basis of inherited disease). La cause génétique de deux de ces maladies, l'abétalipoprotéinémie et l'hypobétalipoprotéinémie a été élucidée. Dans le cas de l'abétalipoprotéinémie, une mutation "frameshift" a été décrite dans le gène de la protéine MTP (Microsomal Triglyceride Transfert) qui conduit à une absence totale de cette protéine et de son activité. Cette mutation empêche par conséquent la formation et la secrétion de lipoprotéines contenant apoB, et donc, la détection d'apoB100 et d'apoB48 dans le plasma des patients (Sharp D et al (1993) Nature, 65-69, Wetterau JR et al (1992) Science, 999-1001). L' hypobétalipoprotéinémie est une maladie dans laquelle différentes mutations dans le gène apoB ont été décrites, conduisant à des protéines apoB tronquées, de différentes tailles. A présent, 25 mutations différentes (nonsense ou frameshift) à l'origine de l'hypobétalipoprotéinémie ont été décrites, résultant en un codon stop prématuré (Linton MF et al (1993) J. Lipid Res., 521-541, Rosseneu M and Lauber C (1995) FASEB J., 768-776). Le mécanisme par lequel ces troncatures de la protéine apoB conduisent à l'hypobétalipoprotéinémie est encore inconnu. Dans le cas de l'abétalipoprotéinémie et de l'hypobétalipoprotéinémie, l'absence d'absorption de chylomicrons conduit également à l'absence d'absorption de vitamine E, créant des symptomes neurologiques sévères.

La troisième de ces maladies génétiques aux phénotypes similaires, la maladie de rétention des chylomicrons, décrite pour la première fois par Anderson, il y a 36 ans (Anderson CM et al (1961) Med. J. Aust., 617-621) reste toujours une énigme. Cette affection est caractérisée par des diarrhées chroniques, une absorption des graisses déficiente, et un manque de vigueur. Dans certains cas, des symptomes neurologiques dus à l'absence de vitamine E ont été observés, mais ceux-ci sont moins sévères que dans le cas de l'abétalipoprotéinémie et de l'hypobétalipoprotéinémie (Havel RJ and Kane JB (1995) in The metabolic and molecular basis of inherited disease). Cette pathologies semble être héritée de façon autosomale récessive. Enfin, l'analyse du plasma des patients montre une absence totale de chylomicrons et de la protéine apoB48 (Havel RJ and Kane JB (1995) in The metabolic and molecular basis of inherited disease).

Des études de liaisons génétiques par RFLP (Restriction Fragment Length Polymorphism) ont montré que le gène apoB n'est pas impliqué dans la maladie d'Anderson (Pessah M et al (1991) J. Clin. Invest., 367-370, Stritch D et al (1993) J. Pediatric Gastro. Nutrit., 257-264). Chez les patients atteints de la maladie d'Anderson, l'activité de la MTP est normale, ce qui suggère qu'un gène différent est impliqué dans cette maladie (Linton MF et al (1993) J. Lipid Res., 521-541). Ces expériences ainsi que d'autres suggèrent donc que l'origine de la maladie d'Anderson n'est pas liée à un problème de secrétion via la MTP, et que la rétention des chylomicrons met en jeu un autre mécanisme (Wetterau JR (1992) Science, 999-1001).

Cette invention concerne des molécules inhibitrices d'apobec-1, ou des protéines associées, susceptibles d'usage thérapeutique dans le cas de l'athérosclérose et de l'obésité, et autres maladies caractérisées notamment par un niveau de chylomicrons et/ou de VLDL dans le plasma supérieur à la normale (hyperlipidémies, comme par exemple hypercholestérolémie, hypertriglycéridémie, etc) et/ou par une hyperglicémie, obtenues à l'aide d'une technique de détection de déamination d'une cytidine dans un ARN (demande de brevet d'invention n° 97 04388) comme décrit dans l'exemple 3. La cytidine déaminée étudiée est ici la cytidine en position 6666 de l'ARNm d'apoB, la séquence d'ARN utilisée comme substrat contenant la zone d'ancrage d'apobec-1 ou des protéines associées est telle que décrit dans la littérature (Shah RR et al (1991) J. Biol. Chem., 16301-16304, Davies MS et al (1989) J. Biol. Chem., 13395-13398), et les extraits de protéines utilisés pourront provenir de foie de rat ou d'autres sources. La séquence utilisée comme amorce dans la mise en oeuvre de la technique contient un nombre de nucléotides complémentaires de la séquence en 3' du site 6666 de l'ARNm d'apoB suffisant pour une hybridation correcte (supérieur ou égal à 14 nucléotides).

Cette invention concerne notamment l'utilisation de molécules anti-sens d'acide nucléique pouvant réduire la quantité d'apobec-1 ou des protéines associées, ou la quantité de protéines exprimés par le ou les gène(s) de la maladie d'Anderson ou tout gène impliqué dans la formation, la stabilisation, la sécrétion, la glycosilation ou le transport des chylomicrons et/ou de VLDL (Uhlmann E and Peyman A (1990) Chem. Rev., 543-584). De telles molécules anti-sens peuvent se lier de façon covalente ou non à l'ADN ou à l'ARN de l'apobec-1 ou des protéines associées, ou à l'ADN ou à l'ARN du ou des gènes responsable(s) de la maladie d'Anderson ou de tout gène impliqué dans la formation, la stabilisation, la sécrétion, la glycosilation ou le transport des chylomicrons et/ou de VLDL. Par exemple, une telle liaison de molécule anti-sens peut cliver ou faciliter le clivage de l'ADN ou ARN, d'apobec-1, ou des protéines associées, ou du ou des gène(s) responsable(s) de la maladie d'Anderson ou de tout gène impliqué dans la formation, la stabilisation, la sécrétion, la glycosilation ou le transport des chylomicrons et/ou de VLDL. Une telle liaison de molécule anti-sens peut également augmenter la dégradation de l'ARNm nucléaire ou cytoplasmique correspondant, ou inhiber sa traduction, la fixation de facteurs de transcription ou d'ARN pré-messager, ou bien encore par exemple inhiber l'épissage de l'ARN pré-messager. L'ensemble de ces modes d'action de molécules anti-sens ont pour effet la diminution de l'expression du gène d'apobec-1, ou des protéines associées, ou du ou des gène(s) responsable(s) de la maladie d'Anderson ou de tout gène impliqué dans la formation, la stabilisation, la sécrétion, la glycosilation ou le transport des chylomicrons et/ou de VLDL, ce qui en fait un mode de traitement important de l'obésité, du diabète de type II (non-insuline dépendant ou de l'athérosclérose, par exemple.

Parmi les cibles potentielles de telles molécules anti-sens, on pourra citer sans pour autant s'y limiter les séquences du gène d'apobec-1, ou des protéines associées, ou du ou des gène(s) responsable(s) de la maladie d'Anderson ou de tout gène impliqué dans la formation, la stabilisation, la sécrétion, la glycosilation ou le transport des chylomicrons et/ou de VLDL, mais également les séquences en 3' et 5' de ces gènes qui pourraient être des régions de contrôle de ces gènes.
La présente invention concerne donc également des molécules anti-sens d'acide nucléique simple brin (ADN ou ARN) d'au moins 12 nucléotides agissant sur le gène ou son ARN, ou sur une région régulatrice de l'expression du gène permettant une ihnibition de l'expression du gène de l'enzyme apobec-1, ou du gène de la protéine ABBP-1 ou de celui d'une protéine associée à l'enzyme apobec-1, ou encore d'un gène impliqué dans la maladie d'Anderson ou de l'activité des enzymes ou protéines exprimées par ces gènes.

### LEGENDES DES FIGURES:

Tableau 1: Trois maladies génétiques possédant des phénotypes similaires. Dans le cas de l'abétalipoprotéinémie, la MTP est mutée, conduisant à l'absence de particules contenant apoB100 ou apoB48 dans le sang. Dans le cas de l'hypobétalipoprotéinémie, le gène apoB est muté, avec pour résultat un niveau très bas de VLDL et de chylomicrons. La maladie d'Anderson se caractérise par l'absence d'apoB48 uniquement, ayant pour conséquence l'absence de chylomicrons à un niveau détectable dans le sang.
Figure 1: Expression d'apoB. Le gène apoB est exprimé dans le foie et l'intestin. Dans le foie, l'ARNm d'apoB est traduit en apoB100, alors que dans l'intestin, le codon CAA commençant à la position 6666 (indiquée par un astérisque) dans l'ARNm est édité par l'enzyme apobec-1, qui déamine la cytidine en uracyl, donnant un codon stop aboutissant à la formation de la protéine apoB48.

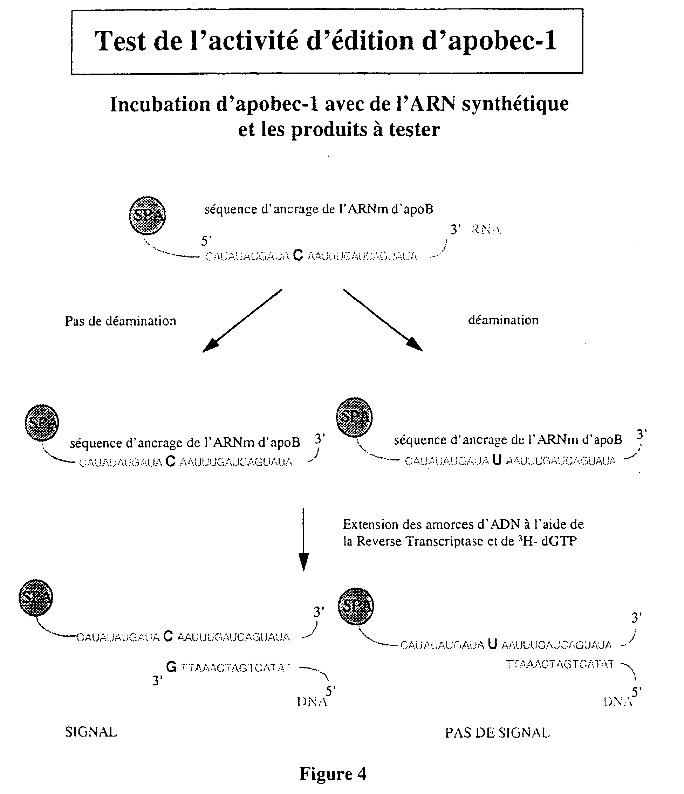

Figure 2: Criblage d'inhibiteurs de l'activité déaminase d'apobec-1 à l'aide d'une technique de détection d'une déamination utilisant le système SPA®. La technique met en jeu une séquence d'ARN synthétique contenant la séquence d'ancrage (environ 50 nucléotides dans le cas d'apobec-1) couplée à une bille SPA®. Après l'incubation avec les inhibiteurs testés en présence de l'enzyme dans le milieu nécessaire, on ajoute une amorce complémentaire de la séquence en 3' du site déaminé de l'ARN dans des conditions d'hybridation. On ajoute alors l'enzyme Reverse Transcriptase et des nucléotides radiomarqués (uniquement d³HGTP dans l'exemple figuré). En cas de déamination, aucune incorporation n'a lieu et le signal détecté ne se distingue pas du bruit de fond. En cas d'activité de l'inhibiteur (pas de déamination), l'incorporation d'un nucléotide radiomarqué a lieu, et un signal est détecté.

### EXEMPLES:

### Exemple 1. Criblage de molécules susceptibles d'inhiber l'activité déaminase d'apobec-1 à l'aide du système SPA®.

L'exemple utilise une séquence d'ARN synthétique contenant la séquence d'ancrage d'apobec-1 (environ 50 nucléotides (Shah RR et al (1991) J. Biol. Chem., 16301-16304, Davies MS et al (1989) J. Biol. Chem., 13395-13398)) couplée à une bille SPA@ (système commercialisé par la société Amersham) disposée dans une plaque de 96 puits, suivant la technique décrite dans la demande de brevet d'invention n° 97 04388. Brièvement, après l'incubation avec les inhibiteurs testés en présence de l'enzyme dans le milieu et avec les réactifs nécessaires, on ajoute une amorce complémentaire de la séquence en 3' du site déaminé de l'ARN dans des conditions d'hybridation (Maniatis T et al (1982) Molecular Cloning, a laboratory manual, Cold Spring Harbor, NY). On ajoute alors une enzyme Reverse Transcriptase et des nucléotides radiomarqués (uniquement d³HGTP dans l'exemple illustré sur la figure 4). Dans le cas présent, il serait également possible d'utiliser d³HTTP en plus. En cas de déamination, c'est-à-dire une activité normale de l'enzyme apobec-1, aucune incorporation n'a lieu et le signal détecté, dû à la scintillation de proximité des billes SPA, ne se distingue pas du bruit de fond. En cas d'activité de l'inhibiteur, l'activité de déamination par apobec-1 est diminuée, rendant possible l'incorporation d'un nucléotide radiomarqué (ou deux si d³HTTP est également utilisé) à l'extrémité 3' d'une partie des amorces hybridées. Dans ce cas, un signal de scintillation peut être détecté qui se distingue du bruit de fond.

**Tableau 1**

| **Maladie** | **Phénotype** | **Diagnostic** | **Déficience** |
|---|---|---|---|
| Maladie d'Anderson | Diarrhée chronique Mauvaise absorption des graisses, Manque de vigueur, Retard de croissance, Symptômes neurologiques modérés | Faible taux de cholestérol et d'apoB100, Taux de triglycéride normal, apoB48 et chylomicrons absents après un repas riche en graisse | ? |
| Abétalipoprotéinémie | Diarrhée chronique Mauvaise absorption des graisses, Manque de vigueur, Retard de croissance, Symptômes neurologiques très sévères | Faible taux de cholestérol, apoB100 et apoB48 absents, Chylomicrons, VLDL, LDL absents, Faible taux de triglycérides et cholestérol | MTP |
| Hypobétalipoprotéinémie | indifférentiable du précédent | Les homozygotes ont un faible taux de cholestérol et de triglycérides, Les hétérozycyotes ont approximativement 50 % du taux de cholestérol, triglycérides et apoB100 | gène apoB tronqué |

## Revendications

1. Procédé de criblage de molécules en vue de l'identification d'agents thérapeutiques destinés au traitement de l'obésité ou du diabète de type II non-insuline dépendant, ledit procédé comprenant la recherche d'inhibiteurs de l'activité de l'enzyme apobec-1 ou de l'expression du gène codant apobec-1.

2. Procédé selon la revendication 1, **caractérisé en ce qu'**il consiste en la recherche d'inhibiteurs de l'activité de l'enzyme apobec-1 ou de l'expression du gène codant apobec-1.

3. Procédé selon la revendication 1 ou 2, **caractérisé en ce que** les inhibiteurs sont recherchés parmi les molécules anti-sens d'acide nucléique simple brin (ADN ou ARN) d'au moins 12 nucléotides agissant sur le gène de l'enzyme apobec-1 ou son ARN, ou sur une région régulatrice de l'expression de ce gène.

## Patentansprüche

1. Verfahren zum Screening von Molekülen in Hinblick auf die Identifizierung von therapeutischen Stoffen, die zur Behandlung von Fettleibigkeit oder nicht insulinabhängigem Diabetes Typ II bestimmt sind, wobei das Verfahren die Suche nach Inhibitoren der Aktivität des Enzyms apobec-1 oder der Expression des für apobec-1 kodierenden Gens umfasst.

2. Verfahren nach Anspruch 1, **dadurch gekennzeichnet, dass** es in der Suche nach Inhibitoren der Aktivität des Enzyms apobec-1 oder der Expression des für apobec-1 kodierenden Gens besteht.

3. Verfahren nach Anspruch 1 oder 2, **dadurch gekennzeichnet, dass** die Inhibitoren unter den Antisens-Molekülen der einsträngigen Nukleinsäure (DNA oder RNA) von mindestens 12 Nukleotiden gesucht werden, die auf das Gen des Enzyms apobec-1 oder seine RNA oder auf eine Regulatorregion der Expression dieses Gens wirken.

## Claims

1. A method of screening of molecules for the identification of therapeutic agents intended for the treatment of obesity or non-insulin dependent type II diabetes, said method comprising the search of inhibitors of the activity of apobec-1 enzyme or of the expression of a gene encoding apobec-1.

2. The method according to Claim 1, **characterized in that** it consists of the search of inhibitors of the activity of apobec-1 enzyme or the expression of a gene encoding apobec-1.

3. The method according to Claim 1 or 2, **characterized in that** the inhibitors are searched among anti-sense single strand nucleic acid molecules (DNA or RNA) of at least 12 nucleotides acting on the gene of apobec-1 enzyme or its RNA, or on a region that regulates the expression of said gene.
